## Europäisches Patentamt
## European Patent Office
## Office européen des brevets

(19)

(11) Veröffentlichungsnummer : **0 058 780**
**B1**

(12) **EUROPÄISCHE PATENTSCHRIFT**

(45) Veröffentlichungstag der Patentschrift :
04.03.87

(21) Anmeldenummer : 81110764.8

(22) Anmeldetag : 24.12.81

(51) Int. Cl.⁴ : **G 01 N 33/53**, G 01 N 33/537,
G 01 N 33/543

(54) Antigen/Antikörper-Bestimmungsmethode.

(30) Priorität : 19.02.81 CH 1104/81
27.11.81 CH 7613/81

(43) Veröffentlichungstag der Anmeldung :
01.09.82 Patentblatt 82/35

(45) Bekanntmachung des Hinweises auf die Patenterteilung : 04.03.87 Patentblatt 87/10

(84) Benannte Vertragsstaaten :
CH DE FR GB IT LI NL

(56) Entgegenhaltungen :
DE-A- 2 907 823
FR-A- 2 273 281
FR-A- 2 359 422
FR-A- 2 418 463
FR-A- 2 433 749
US-A- 4 021 534
US-A- 4 048 298
Die Akte enthält technische Angaben, die nach dem Eingang der Anmeldung eingereicht wurden und die nicht in dieser Patentschrift enthalten sind.

(73) Patentinhaber : **F. HOFFMANN-LA ROCHE & CO.
Aktiengesellschaft**

**CH-4002 Basel (CH)**

(72) Erfinder : **Stocker, John, Dr.
Höhenweg 9
CH-4113 Flüh (CH)**

(74) Vertreter : **Kloter, Rudolf et al
Postfach 3255 Grenzacherstrasse 124
CH-4002 Basel (CH)**

**Beschreibung**

Die Erfindung betrifft ein Verfahren zum qualitativen Bestimmen von Erythrozyten-Antigenen in einer Flüssigkeit durch Inkubieren der Erythrozyten mit Antikörpern bekannter Spezifität.

Das Binden von Antikörpern an Zielantigene, die an Zellen gebunden sind, ist eine grundlegende Reaktion auf allen Gebieten der Serologie und insbesondere der klinischen Diagnostik. Das Bestimmen einer solchen Bindung kann nach einer Vielzahl von Methoden erreicht werden. Diese Methoden umfassen die Beobachtung der Zellagglutination durch die Antikörper, den Zytotoxizitätstest sowie eine Vielfalt von Methoden, bei denen markierte Antikörper verwendet werden, deren Vorhandensein auf der Zelloberfläche durch weitere Methoden aufgedeckt wird, zum Beispiel durch Radioaktivitätsmessung, durch Bestimmung der Enzymsubstratreaktion sowie durch Fluoreszenzmessung.

In der Deutschen Offenlegungsschrift No. 29 07 198 wird eine empfindliche Methode zum Auffinden der Bindung von Antikörpern an Erythrozyten (RBC) beschrieben, welche auf der Adhärenz der Antikörper-beladenen RBC an einer Plastikoberfläche beruht. Bei dieser Methode reagiert das Zielantigen (A), das von den RBC getragen wird, mit einem spezifischen Antikörper von Serum oder Zellen einer Tierspezies X (X-Anti-A). Nach einem Waschvorgang, bei dem überschüssiges X-Anti-A entfernt wird, wird ein weiteres Antiserum (B) zugegeben, das eine Spezifität gegen die Determinanten der Immunglobuline von X-Anti-A aufweist. Nach einem weiteren Wasch-Schritt werden die Zellen in einer Küvette zentrifugiert, deren V-förmiger Boden mit Immunglobulin (Ig) der Tiespezies X (IgX) beladen ist. Die zellgebundenen Antikörper (B) haben freie Bindungsstellen, welche mit dem IgX binden, woraus eine Adhärenz der Zellen an das Plastikmaterial resultiert. Im Fall einer negativen Reaktion, wo kein Anti-A an die Zelloberfläche gebunden ist, bindet Anti-Serum B ebenfalls nicht und es entsteht während der Zentrifugation keine Adhärenz der Zellen. Die in der oben erwähnten Druckschrift beschriebene Methode zeigt zwei Nachteile, nämlich

a) das Erfordernis der mehrmaligen Zellwasch-Schritte, die zeitaufwendig sind, und

b) die Abhängigkeit von einer bestimmten, kritischen Konzentration an X-Anti-A auf der Zelloberfläche, in dem Mass, dass nach wie vor Anti-X Bindungsstellen des zellgebundenen Antiserums (B) vorhanden sind für die Bindung von IgX an die Plastikoberfläche.

Die US-Patentschriften Nos. 4,048,298 und 4,021,534 betreffen klassische Radioimmunverfahren, die auf dem Kompetitionsprinzip basieren. Bei beiden Druckschriften konkurriert das zu suchende Antigen bzw. Hapten mit einer vorgegebenen Menge desselben Antigens bzw. Haptens, welches aber radioaktiv markiert ist, um eine vorgegebene Menge an entsprechenden Antikörpern gegen dieses Antigen. Der entstehende Antigen/Antikörper-Komplex wird dann mit einem zweiten Antikörper, der gegen den ersten Antikörper gerichtet ist, niedergedrückt, was auch mittels Zentrifugation unterstützt werden kann, worauf dann im Niederschlag oder im Ueberstand die Radioaktivität gemessen wird. Aufgrund der gefundenen Menge an Radioaktivität, die mit einer Standardkurve verglichen wird, kann auf die Menge des zu suchenden Antigens bzw. Haptens geschlossen werden.

Gemäss der vorliegenden Erfindung wird ein Antiimmunglobulin oder Protein A direkt an das Plastikmaterial der Küvette gebunden und kann so im Adhärenztest zur Auffindung von X-Anti-A verwendet werden, die an die Zellen gebunden sind. Auf diese Weise werden Wasch-Schritte eliminiert und die Empfindlichkeit des Tests wird erhöht.

Die vorliegende Erfindung betrifft demnach ein Verfahren wie in Anspruch 1 beansprucht.

Als zu bestimmende erythrozytäre Antigene kommen Blutgruppen-Antigene, Rhesus-Antigene, Kell-Antigene, MNS-Antigene, Lewis-Antigene sowie Duffy-Antigene in Betracht.

Die Flüssigkeit, in der die Erythrocyten-Antigen nachgewiesen werden, kann eine biologische Flüssigkeit, wie Blut, Serum oder Plasma sein. Andererseits kann die Flüssigkeit, in der die Erythrocyten-Antigene nachgewiesen werden, auch eine gepufferte Salzlösung, z. B. phosphatgepufferte Kochsalzlösung sein. Im weiteren kommt auch eine Flüssigkeit mit geringer Ionenstärke in Betracht, z. B. eine wässrige Lösung enthaltend 0,24 M/l Glycin, 0,003 M/l $Na_2HPO_4 \cdot 12\ H_2O$, 0,003 M/l $NaH_2PO_4 \cdot 2\ H_2O$, 0,03 M/l NaCl und 0,1 g/l $NaN_3$. Als Behälter mit einer kegel- oder keilförmigen Bodenvertiefung, in dem die Bestimmung von Erythrocytens-Antigenen durchgeführt wird, kommt eine Küvette oder die Vertiefung einer Mikrotiterplatte in Betracht.

Das Anti-Immunglobulin muss nicht unbedingt in gereinigter Form eingesetzt werden, doch ist es von Vorteil, diese in gereinigter Form einzusetzen. Als Reinigungsmethode eignet sich hierzu insbesondere die Affinitätschromatographie.

Nach einem besonderen Aspekt der Erfindung müssen die kegel- oder keilförmigen Bodenvertiefungen der Behälter nicht unmittelbar vor dem Test mit dem Antiimmunglobulin oder Protein A beschichtet werden ; vielmehr ist es möglich, diese kegel- oder keilförmigen Vertiefungen der Behälter mit dem Antiimmunglobulin oder Protein A zu beschichten, mit Wasser auszuwaschen und luftzutrocknen, worauf die so beschichteten Behälter im Kühlschrank bei 4 °C aufbewahrt werden können.

Als Anti-Immunglobuline können solche verwendet werden, die gegen die Immunglobulinsubklassen der Antikörper gerichtet sind. Es ist aber auch möglich, als Anti-Immunglobuline solche zu verwenden, die gegen die leichten Ketten der Antikörper gerichtet sind und somit eine breitere Anwendungsmöglichkeit bieten, da sie gegen alle Immunglobulinklassen gerichtet sind. Die Beschichtung der kegel- oder keilförmigen Bodenvertiefungen der Behälter mit den Anti-Immunglobulinen erfolgt in herkömmlicher Weise.

Die Trennung von ungebundenen Immunglobulinen erfolgt nach dem Einbringen in den Behälter dadurch, dass die den Antigen/Antikörper-Komplex enthaltende Flüssigkeit durch ein Kissen bestehend aus einer weiteren Flüssigkeit zentrifugiert wird, wobei die Dichte der Flüssigkeit des Kissens grösser ist als die Dichte der anderen Flüssigkeit. Selbstverständlich darf die Kissen-Flüssigkeit nicht toxisch sein und muss einen pH im Bereich von 6-8 aufweisen. Im weiteren darf diese Kissen-Flüssigkeit keine Aenderung hinsichtlich der Schichtenfolge bewirken.

Beispiele solcher Kissen-Flüssigkeiten sind Rinderserumalbumin, foetales Kälberserum sowie Polyvinylpyrrolidon.

Wie bereits früher erwähnt, wird nach der Zentrifugation die Menge des Sedimentes bestimmt, wobei dies vorzugsweise durch Messung der ihr proportionalen Sedimentfläche erfolgt.

Die erfindungsgemässe Antigen/Antikörper-Bestimmungsmethode kann sowohl manuell wie auch apparativ, z. B. mit dem in der Deutschen Offenlegungsschrift Nr. 29 07 823 beschriebenen Gerät durchgeführt werden.

Das nachfolgende Beispiel erläutert die Erfindung.

Beispiel

Rhesusbestimmung

1. Herstellung von Kaninchen-Anti-Human IgG (R-Anti-HIgG)

a) Kaninchen werden durch eine erste subkutane Injektion von 0,5 mg humanem IgG in Freund's vollständigem Adjuvans, gefolgt von einer zweiten, vier Wochen späteren Injektion von 0,5 mg IgG in Freund's inkompletten Adjuvans immunisiert. 10 Tage nach der zweiten Injektion werden die Seren mit Hilfe des Ouchterlony Immundiffusionstestes nach Antikörpern gegen humanes IgG untersucht. Kaninchen mit hohen Titern an Antikörpern wird Blut entnommen, und die Seren werden abgetrennt und durch Erhitzen auf 56 °C während 30 Minuten inaktiviert.

b) Die spezifischen Antikörper gegen humanes IgG werden an einer human-IgG-Sepharose Kolonne affinitätschromatographisch gereinigt. Das Antiserum wird auf die Kolonne gebracht, welche dann mit phosphatgepufferter Kochsalzlösung vom pH 7,2 gespült wird. Die spezifischen Antikörper werden dann mit Glycin-Natriumchloridpuffer vom pH 2,8 aus der Säule eluiert. Das Eluat (R-Anti-HIgG) wird dann gegen phosphatgepufferte Kochsalzlösung dialysiert.

2. Beschichtung mit R-Anti-HIgG oder mit Protein A

a) Die R-Anti-HIgG bzw. das Protein A (Pharmacia) werden in phosphatgepufferter Kochsalzlösung auf 10 µg/ml verdünnt. 50 µl werden zu jeder Vertiefung einer Mikrotiterplatte mit V-förmigem Boden (Dynatech Laboratories Cat. No. 1-330-25, Virginia, USA) gegeben. Die Platten werden während einer Stunde bei 37 °C inkubiert.

b) Die Flüssigkeit mit phosphatgepufferter Kochsalzlösung und ungebundenem R-Anti-HIgG oder Protein A wird entfernt und die Mikrotiterplatten werden zweimal mit phosphatgepufferter Kochsalzlösung gewaschen und dann kurz mit destilliertem Wasser gewaschen.

c) Das destillierte Wasser wird sofort entfernt, und die Mikrotiterplatten werden in einem kühlen Luftstrom luftgetrocknet. Die getrockneten Platten können dann bei 4 °C bis zur Verwendung gelagert werden.

3. Bestimmung von RBC-Antigenen unter Verwendung von mit R-Anti-HIgG oder mit Protein A beschichteten Platten

a) 100 µl Citrat-antikoaguliertes

Spenderblut werden durch Zugabe von 5 ml eines Puffers mit geringer Ionenstärke verdünnt. Als Puffer mit geringer Ionenstärke wird 4 %ige Glukose in Wasser mit 5 mM HEPES (N-2-Hydroxyäthylpiperazin-N'-2-äthansulfonsäure) Puffer verwendet.

b) 50 µl des verdünnten Blutes werden mit 10 µl Rhesusblutgruppen-Antiserum (Merz und Dade, Düdingen, Schweiz) während 10 Minuten bei Raumtemperatur inkubiert.

c) In jede Vertiefung der Mikrotiterplatte, die mit R-Anti-HlgG oder mit Protein A beschichtet ist, werden 150 µl 20 %iges Rinderserumalbumin in phosphatgepufferter Kochsalzlösung gegeben.

d) 25 µl des Inkubationsgemisches gemäss 3b) werden auf die Oberfläche der phosphatgepufferten Kochsalzlösung überschichtet.

e) Die Platten werden während 5 Minuten bei 3 000 rpm in einer Zentrifuge mit einem Rotationsradius von 11 cm zentrifugiert (dies entspricht ungefähr 1 100 × g).

f) Das Ausmass des Erythrozytensedimentes wird visuell abgeschätzt. Im Fall, wo weniger als 10 % der Erythrozyten im Sediment enthalten sind, wird die Reaktion als positiv (+) beurteilt, was bedeutet, dass die Erythrozyten das Antigen tragen, welches von dem verwendeten Antiserum erkannt wird. Im Falle, wo mindestens 70 % der Erythrozyten im Sediment vorhanden sind, wird der Test als negativ betrachtet (—). Bei Verwendung rhesustypischer Antiseren wurden keine Zwischenresultate beobachtet.

4. Ergebnis

a) Die Rhesusbestimmung gemäss der erfindungsgemässen Festphasenmethode (SPA) wird mit der herkömmlichen Coombs Antiglobulinmethode (CT) verglichen. Die Ergebnisse sind in der folgenden Tabelle gezeigt.

(Siehe Tabelle Seite 5f.)

Tabelle

| Spezifität des rhesus-typischen Serums | Testresultate | | | | | | | |
|---|---|---|---|---|---|---|---|---|
| | Spender No. 1 | | | Spender No. 2 | | | Spender No. 3 | |
| | CT | SPA | | CT | SPA | | CT | SPA |
| | | R-Anti-HIgG | Protein A | | R-Anti-HIgG | Protein A | | R-Anti-HIgG |
| C | − | − | − | − | − | − | + | + |
| C | + | + | + | + | + | + | + | + |
| E | + | + | + | − | − | − | − | − |
| e | + | + | + | + | + | + | + | + |
| D | + | + | + | − | − | − | + | + |
| Negative Kontrolle (phosphatge - pufferte Kochsalz- lösung | − | − | − | − | − | − | − | − |

0 058 780

**Patentansprüche**

1. Verfahren zum qualitativen Bestimmen von Erythrozyten-Antigenen in einer Flüssigkeit durch Inkubieren der Erythrozyten mit Antikörpern bekannter Spezifität, dadurch gekennzeichnet, dass man die den Antigen/Antikörper-Komplex enthaltende Flüssigkeit in einen Behälter mit kegel- oder keilförmiger Bodenvertiefung einbringt, wobei mindestens die Vertiefung des Behälters mit Antiimmunglobulin oder Protein A beschichtet ist, welche gegen die Antikörper gerichtet ist, und dass man die den Antigen/Antikörper-Komplex enthaltende Flüssigkeit durch ein Kissen bestehend aus einer weiteren, nicht toxischen Flüssigkeit vom pH 6-8 zentrifugiert, wobei die Dichte der Flüssigkeit des Kissens grösser ist als die Dichte der anderen Flüssigkeit, und dass man nach der Zentrifugation die Menge des Sediments bestimmt, wobei dies durch Messung der ihr proportionalen Sedimentfläche erfolgt.

2. Verfahren nach Anspruch 1, dadurch gekennzeichnet, dass die Flüssigkeit eine biologische Flüssigkeit ist.

3. Verfahren nach Anspruch 1, dadurch gekennzeichnet, dass die Flüssigkeit gepufferte Salzlösung ist.

4. Verfahren nach Anspruch 1, dadurch gekennzeichnet, dass die Flüssigkeit ein Puffer mit geringer Ionenstärke ist.

5. Verfahren nach Anspruch 1, dadurch gekennzeichnet, dass der Behälter mit kegel- oder keilförmiger Bodenvertiefung eine Küvette oder die Vertiefung einer Mikrotiterplatte ist.

6. Verfahren nach Anspruch 1, dadurch gekennzeichnet, dass man die kegel- oder keilförmigen Bodenvertiefungen der Behälter mit der Immunglobulin-bindenden Komponente beschichtet, mit Wasser auswäscht und lufttrocknet.

**Claims**

1. A method for the qualitative determination of erythrocytic antigens in a fluid by incubating the erythrocytes with antibodies of known specificity, characterized by introducing the fluid containing the antigen/antibody complex into a container having a conical-shaped or keel-shaped bottom recess, whereby at least the recess of the container is coated with antiimmunoglobulin or protein A which is directed against the antibody, and centrifuging the fluid containing the antigen/antibody complex through a cushion consisting of a further, non-toxic fluid of pH 6-8, whereby the density of the cushion fluid is greater than the density of the other fluid, and, after the centrifugation, determining the amount of the sediment, whereby this is effected by measuring its proportional sediment surface.

2. A method according to claim 1, characterized in that the fluid is a biological fluid.

3. A method according to claim 1, characterized in that the fluid is buffered salt solution.

4. A method according to claim 1, characterized in that the fluid is a buffer having a low ion strength.

5. A method according to claim 1, characterized in that the container having a conical-shaped or keel-shaped botton recess is a cuvette or the well of a microtitre plate.

6. A method according to claim 1, characterized in that the conical-shaped or keel-shaped bottom rescesses of the container are coated with the immunoglobulin-binding component, washed out with water and air-dried.

**Revendications**

1. Procédé pour la détermination qualitative des antigènes des érythrocytes dans un fluide par incubation des érythrocytes avec des anticorps de spécificité connue, caractérisé en ce que l'on introduit le fluide contenant le complexe antigène/anticorps dans un récipient ayant dans le fond une partie creuse de forme conique ou en coin, la partie creuse au moins du récipient étant revêtue d'une anti-immunoglobuline ou d'une protéine A dirigée contre les anticorps, et en ce que l'on centrifuge le fluide contenant le complexe antigène/anticorps au travers d'un coussin consistant en un autre fluide non toxique à pH 6 à 8, la densité du fluide du coussin étant supérieure à la densité de l'autre fluide, et après centrifugation, on détermine la quantité du sédiment par mesure de sa surface qui est proportionnelle à sa quantité.

2. Procédé selon la revendication 1, caractérisé en ce que le fluide est un fluide biologique.

3. Procédé selon la revendication 1, caractérisé en ce que le fluide est une solution saline tamponnée.

4. Procédé selon la revendication 1, caractérisé en ce que le fluide est un tampon à faible force ionique.

5. Procédé selon la revendication 1, caractérisé en ce que le fond du récipient ayant une partie creuse de forme conique ou en coin est une cuvette ou la partie creuse d'une plaque de microtitrage.

6. Procédé selon la revendication 1, caractérisé en ce que l'on revêt les parties creuses de forme conique ou en coin du fond du récipient par le composant fixant l'immunoglobuline, on lave à l'eau et on sèche à l'air.